## Europäisches Patentamt

## European Patent Office

## Office européen des brevets

(19)

(11) Publication number: **0 239 644**

**A1**

(12) # EUROPEAN PATENT APPLICATION

published in accordance with Art. 158(3) EPC

(21) Application number: **86904354.7**

(22) Date of filing: **27.06.86**

Data of the international application taken as a basis:

(86) International application number:
**PCT/JP86/00330**

(87) International publication number:
**WO87/00050 (15.01.87 87/01)**

(51) Int. Cl.³: **A 61 K 35/42**
**A 61 K 37/02, C 07 K 15/06**

(30) Priority: **28.06.85 JP 140139/85**

(43) Date of publication of application:
**07.10.87 Bulletin 87/41**

(84) Designated Contracting States:
**CH DE FR GB IT LI SE**

(71) Applicant: **Asahi Kasei Kogyo Kabushiki Kaisha**
**2-6, Dojimahama 1-chome Kita-ku**
**Osaka-shi Osaka 530(JP)**

(72) Inventor: **SUZUKI, Koji**
**191-2, Torii-cho**
**Tsu-shi Mie-ken 514(JP)**

(72) Inventor: **YAMAMOTO, Shuji**
**168-27, Obuchi Fuji-shi**
**Shizuoka-ken 417(JP)**

(74) Representative: **Boeters, Hans Dietrich, Dr. et al,**
**Boeters, Bauer & Partner Thomas-Wimmer-Ring 14**
**D-8000 München 22(DE)**

(54) **NOVEL PHYSIOLOGICALLY ACTIVE SUBSTANCE HAVING BLOOD COAGULATION CONTROLLING ACTIVITY.**

(57) A novel physiologically active substance having an amino acid sequence of Pro-Ala-Glu-Pro-Gln-Pro-Gly-Gly-Ser-Gln- as an amino acid sequence starting from the second amino acid from the amino terminal. The active substance is obtained from human lung and has an anti-coagulating effect, an effect of preventing agglutination of thrombocytes, and an effect of dissolving thrombus without causing hemorrhage or anaphylaxy shock, thus being effectively usable for treating various diseases of circulatory organs.

FIG. 2

## Specification

### Field of the Invention

The present invention relates to a novel physiologically active substance capable of controlling blood coagulation. More particularly, the present invention is concerned with thrombomodulin derived from human (hereinafter often referred to as "human thrombomodulin") which has thrombolytic, anticoagulant and platelets aggregation-inhibiting activities and is useful for the treatment of circulatory organ diseases.

### Description of the Prior Art

Thrombolytic drugs presently in use include streptokinase and urokinase, and plasminogen activator is now being developed. As an anticoagulant, heparin and warfarin are in use. As a drug for inhibiting the aggregation of blood platelets, aspirin, sulfinpyrazone, dipyridamole and the like are used.

Nowadays, these thrombolytic drugs, anticoagulant drugs and drugs for inhibiting the aggregation of blood platelets are used, alone or in combination, for the treatment and prevention of circulatory organ diseases such as myocardial infarction, thrombosis, embolism, obstruction of peripheral blood vessels, arteriosclerosis obliterans, disseminated intravascular coagulation (DIC) syndrome, angina pectoris and transient ischemic attack.

On the other hand, protein C is known as a protein which is dependent on vitamin K and plays an important role in the blood coagulation mechanism. In the recent years, a substance which greatly accelerates the activation of the protein C was isolated from rabbit lung and purified. The substance was named thrombomodulin [N.L. Esmon et al., J. Biol. Chem., Vol. 257, p.859 (1982)]. It is known that in the living body, activated protein C inactivates activated factors V and VIII of the blood coagulation system coenzymes, and takes part in the production of plasminogen activator which has thrombolytic activity [Kohji Suzuki, Igaku No Ayumi (History of Medicine), Vol. 125, p.901 (1983)]. Protein C is activated by thrombin into activated protein C and exhibits anticoagulant and thrombolytic activities. If thrombomodulin is present as a cofactor of thrombin in the system of activation of protein C, the production of activated protein C is remarkably accelerated [C.T. Esmon et al., Proc. Natl. Acad. Sci. USA, Vol. 78, p.2249 (1981)]. That is, thrombomodulin accelerates the activation of protein C, thereby leading to the production of a large quantity of activated protein C which exhibits anticoagulant and thrombolytic activities. Hence, thrombomodulin greatly contributes to in vivo anticoagulation and thrombolysis.

It should be noted here that although thrombin is capable of activating protein C as mentioned above, it also has activities of activating fibrinogen into fibrin and aggregating blood platelets.

Thrombomodulin is strongly bonded with thrombin and directly inhibits the above-mentioned activities of thrombin [ C.T. Esmon et al., J. Biol. Chem., Vol. 257, p.7944 (1982); and N.L. Esmon et al., J. Biol. Chem., Vol. 258, p.12238 (1983)]. Illustratively stated, thrombomodulin not only has thrombolytic activity but also inhibits blood coagulation through inhibition of the formation of fibrin, namely, has anticoagulant activity, and inhibits the aggregation of blood platelets. Further, an albuminous substance derived from human placenta which has activities similar to those of the above-mentioned thrombomodulin was recently reported [Japanese Patent Application Laid-Open Specification No. 60-199819; and H.H. Salem et al., J. Biol. Chem., Vol. 259, p.12246 (1984)]. However, the substance is not purified enough to determine the amino acid sequence of the substance. Drugs used nowadays for the treatment of circulatory organ diseases, such as thrombolytic drugs, anticoagulant drugs and drugs for inhibiting the aggregation of blood platelets are often administered in combination. Moreover, to exert necessary effects in the treatment, these drugs have to be administered in large dosages. Therefore, side effects such as bleeding may occur. Under these circumstances, a drug is desired which is effective with a small dosage and has not only anticoagulant and platelets aggregation-inhibiting activities but also thrombolytic activity even if it is used alone.

In this respect, the above-mentioned rabbit thrombomodulin, which has already been reported, is expected to become an epoch-making medicine. However, if the rabbit thrombomodulin is administered to a human body as a drug for the treatment of circulatory organ diseases, it is possible that the so-called anaphylactic shock occurs. The reason is that since thrombomodulin contains peptides, if thrombomodulin is administered to an animal other than the animal from which the thrombomodulin is derived, the thrombomodulin acts as an antigen and causes an antibody to be produced. For this reason, if thrombomodulin is to be administered to a human body, it is greatly preferred that thrombomodulin derived from human be used. However, human thrombomodulin in a highly purified form has not been obtained yet.

## Disclosure of the Present Invention

It is an object of the present invention to provide human-derived thrombomodulin which has anticoagulant activity, platelets aggregation-inhibiting activity, thrombolytic activity and the like.

According to the present invention, there is provided a physiologically active substance capable of controlling blood coagulation, which is characterized by the following properties:

(a)　molecular weight:

105,000 ± 10,000 (as measured by SDS-polyacrylamide gel electrophoresis after reduction);

(b)　stability:

(1) stable at 60 °C for 30 min,

(2) not inactivated at 100 °C for 10 min,

(3) stable at pH 2 to 10,

(4) stable with 1 % SDS treatment,

(5) stable with 6 M urea treatment,

(6) not inactivated with 6 M guanidine hydrochloride,

(7) not inactivated with 3 M KSCN treatment, and

(8) inactivated with 1 % 2-mercaptoethanol treatment;

(c)　activities:

(1) it activates protein C as a cofactor of thrombin,

(2) it inhibits the conversion of fibrinogen to fibrin by thrombin, thereby substantially inhibiting blood coagulation, and

(3) it substantially inhibits the platelets aggregation activity of thrombin; and

(d)   chemical structure:

it contains a peptide having the following amino acid sequence as from the second amino acid from the amino terminal

Pro-Ala-Glu-Pro-Gln-Pro-Gly-Gly-Ser-Gln.

(wherein Pro represents a proline residue, Ala an alanine residue, Glu a glutamic acid residue and Gln a glutamine residue, Gly a glycine residue and Ser a serine residue).

The physiologically active substance according to the present invention is characterized in that it has the following amino acid sequence as from the second amino acid from the amino terminal:  Pro-Ala-Glu-Pro-Gln-Pro-Gly-Gly-Ser-Gln- (wherein Pro represents a proline residue, Ala an alanine residue, Glu a glutamic acid residue, Gln a glutamine residue, Gly a glycine residue and Ser a serine residue). The amino acid at the amino terminal of the physiologically active substance according to the present invention is alanine, serine or glycine.

The physiologically active substance of the present invention is also characterized in that the substance has a molecular weight of $105,000 \pm 10,000$ in terms of a value measured by SDS-polyacrylamide gel electrophoresis after reduction and exhibits various chemical and physical stabilities as mentioned above.

Further, the physiologically active substance of the present invention is characterized by abilities to (1) activate protein C as a cofactor of thrombin; (2) inhibit the conversion of fibrinogen to fibrin by thrombin, thereby substantially inhibiting blood coagulating activity; and (3) substantially inhibit the platelets aggregation activity of thrombin.

The physical and chemical properties of the physiologically active substance according to the present invention may be examined by the methods as explained below. The activity of the physiologically active substance of the present invention was measured according to the known protein C assay method in which use is made of a synthetic substrate having the formula Boc-Leu-Ser-Thr-Arg-MCA (Boc and MCA are abbreviations of t-butoxycarbonyl group and 4-methylcoumaryl-7-amide, respectively.) [Y. Ohno et al, J. Biochem., Vol. 90, p.1387 (1981)]. Illustratively stated, to a reaction liquor containing 0.5μM protein C and 80 nM thrombin are added 5 μl of an aqueous solution of the substance of the present invention (0 to 0.01 $A_{280}$/ml), and then added 20 mM Tris-HCl buffer (pH 7.4) containing 0.15 M NaCl, 2.5 mM $CaCl_2$ and 1 mg/ml bovine serum albumin so that the whole volume becomes

30 μℓ. The obtained mixture is subjected to a reaction at 37 °C for 15 min to activate protein C. Then, 10 μℓ of an aqueous solution containing 10 μℓ of 2 μM antithrombin III and 10 U/mℓ heparin is added to the reaction mixture and the resultant mixture is heated at 37 °C for 15 min so as to terminate the reaction. To the resultant mixture is added 250 μℓ of 20 mM Tris-HCl buffer (pH 7.4) containing 200 μM Boc-Leu-Ser-Thr-Arg-MCA which is the synthetic substrate as mentioned above [manufactured by Peptide Institute, Inc. (Osaka, Japan)] and the mixture containing the substrate is subjected to a reaction at 37 °C for 10 min. Then, 0.5 mℓ of 20 % acetic acid is added to the mixture to terminate the reaction. The concentration of free AMC (7-amino-4-methyl-coumarin) is measured by a fluorescence spectrophotometer using an exciting wavelength of 380 nm and an emission wavelength of 440 nm. The obtained fluorescence is compared with the fluorescence intensity of AMC having a known concentration to determine the amount of the free AMC. Since this amount of the free AMC depends on the strength of the thrombomodulin activity in the sample, thrombomodulin activity is expressed in terms of the amount of AMC produced per minute.

In effecting the assay, protein C was purified from human blood plasma by the method of Suzuki et al. [Suzuki et al., J. Biol. Chem., Vol. 258, p.1914 (1983)]. On the other hand, human thrombin was purified by the method of Lundblad et al. [Lundblad et al., Biochem. Biophys. Res. Commun. Vol. 66, p. 482 (1975)].

(a)  Method for determining molecular weight

According to the method of U.K. Laemmli [Nature, Vol. 227, p. 680, (1970)], a sample treated with 2-mercaptoethanol is subjected to SDS-polyacrylamide gel electrophoresis. A molecular weight calibration curve is obtained using a standard molecular weight kit (produced by Pharmacia Fine Chemicals AB, Sweden). On the other hand, the migration position of the active substance of the present invention is examined by the activity assay and staining with Coomassie Brilliant Blue R-250. Then, the migration position is compared with the above-mentioned molecular weight calibration curve to determine the molecular weight.

(b)  Method for testing stability

(1)   0.02M Tris-HC$\ell$ buffer (pH 7.5) containing the physiologically active substance of the present invention in a concentration of 0.006 $A_{280}$/m$\ell$ (concentration at which the absorbance at a wavelength of 280 nm is 0.006 per m$\ell$) and 0.1M of salt is heated at 60 °C for 30 min.

(2)   The same buffer as described in (1) above is prepared and then heated at 100 °C to determine the time required for inactivating 50 % of the activity.

(3)    The substance of the present invention is added, in a concentration of 0.09 $A_{280}$/ml, to a solution containing 0.1M NaCl, 0.02M glycine, 1 mM benzamidine hydrochloride, 0.1 %(v/v) Triton X-100 and 5 mM $CaCl_2$ and having a pH of 2, and treated with the solution for a period of 2.5 hours. Separately, the substance of the present invention is also treated with a solution having the same composition as mentioned above but a pH of 10.

(4)    The substance of the present invention is added, in a concentration of 0.007 $A_{280}$/ml, to 0.02M Tris-HCl buffer (pH 7.5) containing 1 % (w/v) SDS (sodium lauryl sulfate) and 0.1M NaCl, and treated with the buffer at room temperature for 2.5 hours. Then, the solution containing the substance is diluted 100-fold with 0.02M Tris-HCl buffer (pH 7.5) containing 0.1M NaCl and 1 mg/ml bovine serum albumin and immediately subjected to assay of the activity.

(5)    Substantially the same treatment as described in (4) above is conducted except that 6M urea is used instead of 1 % (w/v) SDS.

(6)    Substantially the same treatment as described in (4) above is conducted except that 6M guanidine hydrochloride is used instead of 1 % (w/v) SDS.

(7)    Substantially the same treatment as described in (4) above is conducted except that 3M KSCN is used instead of 1 % (w/v) SDS.

(8)　Substantially the same treatment as described in (4) above is conducted except that 0.1 % (v/v) 2-mercaptoethanol and 1.0 % (v/v) 2-mercaptoethanol are used, respectively, instead of 1 % (w/v) SDS, thereby to examine the effect of mercaptoethanol on the physiologically active substance.

(c)　Method for evaluating activities

(1)　The following method is used to evaluate the activity of the substance of the present invention as a cofactor of thrombin for the activation of protein C.

That is, to 0.02M Tris-HC$l$ buffer (pH 7.5) containing 0.1M NaC$l$, 3.6 mM CaC$l_2$ and 10 mg/m$l$ bovine serum albumin are added 50 μg/m$l$ protein C, 5 nM thrombin and 5 nM thrombomodulin and the resulting mixture is subjected to a reaction at 37 °C. The reaction is terminated with 300 μg/m$l$ antithrombin III (manufactured by Sigma Chemical Company, U.S.A.) and 5 mM EDTA. Then, the amount of the activated protein C produced in the reaction is measured by the abovementioned method in which a synthetic substrate is used.

(2)　The inhibiting effect of the substance of the present invention on the conversion of fibrinogen to fibrin by thrombin, which leads to substantial inhibition of the blood coagulation, is examined by measuring the blood coagulation time using Coagulometer KC-10 manufactured by Heinrich Amelung A.G. (West Germany). That is, to 0.05M Tris-HC$l$ buffer (pH

7.5) containing 5 mM CaCl$_2$ and 0.1M NaCl is added 30 μg of fibrinogen (Fraction I, manufactured by Sigma Chemical Company, U.S.A.). To the resulting mixture are added 0 to 50 nM thrombomodulin solutions. Then, 10 nM thrombin is added to the above-obtained mixture in such an amount that the total amount of the resultant mixture becomes 0.4 ml, and the coagulation time is measured.

(3) The effect of the substance of the present invention with respect to substantial inhibition of the platelets-aggregating activity of thrombin is evaluated using Platelet Aggregometer manufactured by SIENCO Co., Ltd. (U.S.A.). It is known that when 1 unit of thrombin (about 0.4 μg) is added to 250 μl of 300,000 cells/μl platelet rich plasma (P.R.P.), the platelets aggregate. Before the addition of thrombin the physiologically active substance of the present invention is added to the plasma, and the platelets aggregation reaction is observed.

(d) Chemical structure

The amino acid sequence of the physiologically active substance of the present invention is analyzed as follows.

Purified human thrombomodulin is dialyzed against a 0.1 % (v/v) aqueous solution of sodium lauryl sulfate (SDS) at room temperature for 16 hours to obtain a sample for the analysis of the amino acid sequence. Using an amino acid sequencing analyzer (Model 470 A) manufactured by Applied Biosystems Inc. (U.S.A.), Edman degradation is effected successively starting from the N-terminus according to the method of R.M. Hewick et al [J. Biol. Chem., Vol. 256, p. 7990 (1981)]. The liberated phenylthiohydantoin amino acid is analyzed using an apparatus for high speed liquid chromatography (SP-8100) manufactured by Spectra Physics (U.S.A.) and Solpacks ODS column manufactured by E.I. du Pont de Nemours and Company, U.S.A. to determine the amino acid sequence.

The physiologically active substance of the present invention may be obtained by extraction from human lung and purification of the extract. As the human lung, there may be employed a lung which is obtained through an autopsy of the body of a human adult who died by a cause other than pneumopathy, i.e. a human lung having normal tissues. The physiologically active substance of the present invention may be extracted from human lung by cutting a lung into fragments, homogenizing the fragments, and then subjecting them to extraction with an appropriate solvent. The obtained extract is subjected to purification by a known customary method to obtain a purified physiologically active substance according to the present invention. As the method for purifying the physiologically active substance of the present invention, there may be mentioned those which are generally used for the purification of proteins such as an adsorption method using a carrier, an ion-exchange method, a fractional precipitation method, a gel-filtration method, an electrophoresis, various affinity chromatographies and a method in which a specific antibody is used. These methods may be used alone or in combination.

Illustratively stated, the extraction and purification may be effected, for example, by the following method. First, a human lung is treated in a homogenizer and the obtained pellets are treated with a surface active agent such as Triton X-100 to liberate and extract thrombomodulin.

Subsequently, the obtained extract is passed through a diisopropyl phosphoro-thrombin (hereinafter often referred to as DIP-thrombin) agarose column. After washing the column with a rinsing liquid containing 0.3M NaCℓ, the extract is eluted with an eluent containing 1M NaCℓ and dialyzed. After the dialysis, the dialyzate is further passed through the DIP-thrombin-agarose column. After washing the column with a rinsing liquid containing 0.4M NaCℓ, the extract is eluted with an eluent containing 1M NaCℓ, and dialyzed. After the dialysis, the purification using the DIP-thrombin-agarose column is further repeated twice under the same conditions as in the second purification. In this way, the physiologically active substance of the present invention can be obtained in a substantially pure form.

The physiologically active substance of the present invention has anticoagulant, platelets aggregation-inhibiting and thrombolytic activities and, hence, can be used, for example, for the treatment and prevention of circulatory organ diseases such as myocardial infarction, thrombosis, embolism, obstruction of peripheral blood vessels, arteriosclerosis obliterans, disseminated intravascular coagulation (DIC) syndrome, angina pectoris and transient ischemic attack. For the treatment of the above-mentioned diseases the physiologically active substance of the present invention may be used in the form of a mixture with a pharmaceutically employable carrier. That is, an effective amount of the physiologically active substance of the present invention may be mixed with an appropriate amount of a carrier to prepare a pharmaceutical composition which is suitable for effective administration to the patient.

The physiologically active substance of the present invention may be used in the form of an injection etc. If the substance is used in the form of an injection, there may be added, as an additive, a thickening agent such as sucrose, glycerin, methyl cellulose or carboxymethylcellulose, a pH adjuster of various inorganic salts, or the like.

The dose of the physiologically active substance of the present invention for an adult varies with the age, sex, weight, severities of disorders, etc. of the patient. In general, however, the dose is about 0.1 to about 200 mg. The present substance may be administered once a day or, if necessary, several times a day.

## Preferred Embodiment of the Invention

The present invention will be described in more detail with reference to the Examples, which should not be construed to be limiting the scope of the present invention.

Example 1

About 800 g of a human lung obtained through an autopsy was cut with scissors into small squares of about 1 cm x 1 cm. To the obtained tissue fragments was added 500 m$\ell$ of physiological saline containing 1 mM DFP (diisopropyl fluorophosphate) which had been cooled to 4 °C. The obtained mixture was homogenized using a Waring blender at 4 °C for 5 min. After the homogenization, the mixture was cooled in ice for 5 min. Then, the mixture was further homogenized at 4 °C for 5 min and cooled in ice for 5 min. The above-described homogenizing and cooling operations were further repeated thrice. The obtained homogenate was subjected to centrifugation at 12,000 g at 4 °C for 30 min to separate the homogenate into a supernatant and pellets, and the pellets were collected. The collected pellets were suspended in 100 m$\ell$ of 0.02M Tris-HC$\ell$ buffer (pH 7.5) containing 0.5 % (v/v) Triton X-100, 0.25M sucrose, 1 mM benzamidine hydrochloride and 0.5 mM CaC$\ell_2$, and subjected 5 times to homogenization at 4 °C for 5 min to effect extraction.

The obtained extract was subjected to centrifugation at 35,000 g at 10 °C for 60 minutes, and the supernatant was collected. DIP-thrombin (diisopropylphosphorothrombin), which had been prepared according to the method of N.L. Esmon et al [J. Biol. Chem., Vol. 257, p.859 (1982)], was bonded to an agarose which had been treated with cyanogen bromide according to the method of P. Cuatrecasas [J. Biol. Chem., Vol. 245, p.3059 (1970)], thereby to prepare DIP-thrombin-agarose.

Then, the DIP-thrombin-agarose was packed in a 2.5 cm∅ x 10 cm column to make up a column of DIP-thrombin-agarose. The column was equilibrated at room temperature with 0.02 M Tris-HCl buffer (pH 7.5) containing 0.1 M NaCl, 0.5 mM $CaCl_2$, 1 mM benzamidine-hydrochloric acid and 0.5 % (v/v) of Lubrol PX (produced and sold by Nakarai Chemical Ltd., Japan). Subsequently, the above-mentioned supernatant of the extract was passed through the column. The column was washed with 0.02 M Tris-HCl buffer (pH 7.5) containing 0.3 M NaCl, 0.5 mM $CaCl_2$, 1 mM benzamidine-hydrochloric acid and 0.5 % (v/v) of Lubrol PX, followed by elution with 0.02 M Tris-HCl buffer (pH 7.5) containing 1 M NaCl, 0.1 mM EDTA, 1 mM benzamidine-hydrochloric acid and 0.5 % (v/v) of Lubrol PX. With respect to each of the fractions obtained through the elution, the thrombomodulin activity was determined by the method as described hereinbefore. At the same time, the absorbance ($A_{280}$) of each fraction was determined at 280 nm by means of a spectrophotometer (model UV-240) manufactured and sold by Shimadzu Corp., Japan.

Fractions having thrombomodulin activity were recovered and pooled, and dialyzed against 0.02 M Tris-HCl buffer (pH 7.5) containing 0.1 M NaCl, 0.5 mM $CaCl_2$ and 0.05 % (v/v) of Lubrol PX. The dialyzate obtained was subjected to the second DIP-thrombin-agarose column chromatography as follows. The dialyzate was passed through a 1.5 cm$\phi$ x 10 cm column of DIP-thrombin-agarose. The column was washed with 0.02 M Tris-HCl buffer (pH 7.5) containing 0.4 M NaCl, 0.5 mM $CaCl_2$ and 0.1 % (v/v) of Lubrol PX, and further washed with 0.02 M Tris-HCl buffer (pH 7.5) containing 0.4 M NaCl, 0.1 mM EDTA and 0.1 % (v/v) of Lubrol PX, followed by elution with 0.02 M Tris-HCl buffer (pH 7.5) containing 1 M NaCl, 0.5 mM EDTA and 0.1 % (v/v) of Lubrol PX. Fractions having thrombomodulin activity were recovered and pooled, and further dialyzed against 0.02 M Tris-HCl buffer (pH 7.5) containing 0.1 M NaCl and 0.05 % (v/v) of Lubrol PX. The dialyzate thus obtained was passed through a column of DIP-thrombin-agarose for the third column chromatography. The size of the column and the conditions of washing and elution were the same as those in the second DIP-thrombin-agarose column chromatography. Through the elution, fractions each of 2 ml were collected.

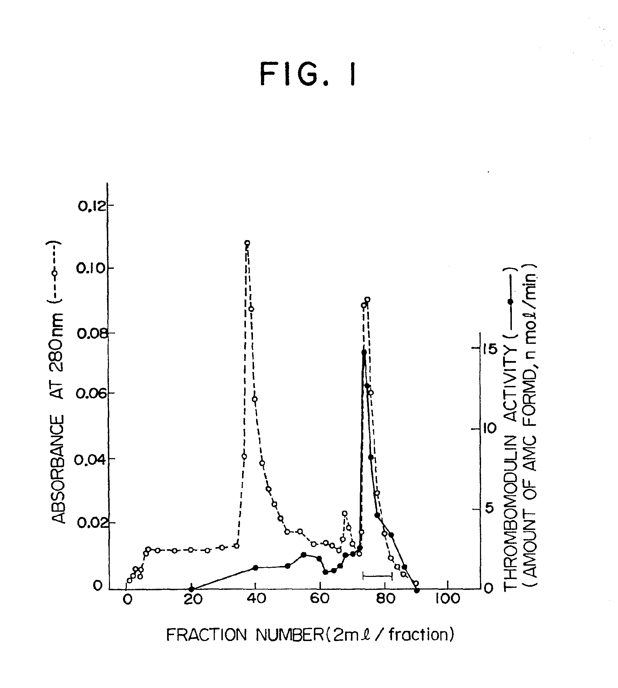

Fig. 1 shows the elution patterns of the third DIP-thrombin-agarose column chromatography. The fractions of Nos. 72 to 82 were recovered and pooled, and dialyzed against 0.02 M Tris-HCl buffer (pH 7.5) containing 0.1 M NaCl and 0.05 % (v/v) of Lubrol PX. Then, the dialyzate was passed through a 0.9 cm$\phi$ x 8 cm column of DIP-thrombin-agarose. The column was washed with 0.02 M Tris-HCl buffer (pH 7.5) containing 0.35 M NaCl, 0.5 mM $CaCl_2$ and 0.1 % (v/v) of Lubrol PX, followed by elution with 0.02 M Tris-HCl buffer (pH 7.5) containing 1 M NaCl, 0.5 mM EDTA and 0.1 % (v/v) of Lubrol PX. Through the elution, fractions each of 1.9 m$l$ were collected.

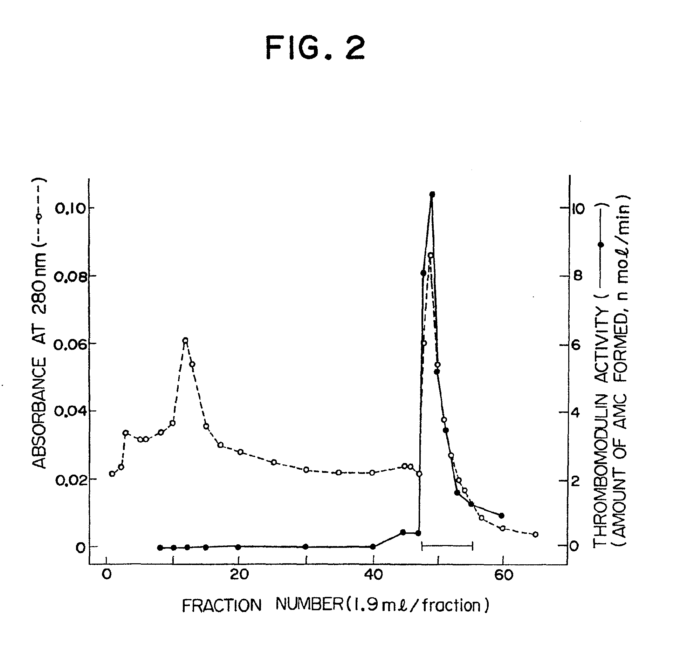

Fig. 2 shows the elution patterns of the fourth DIP-thrombin-agarose column chromatography. The fractions of Nos. 48 to 56 were recovered and pooled.

The molecular extinction coefficient for general proteins, which is $10.0 (E_1^{1\ \%}{}_{cm},\ 280\ nm=10.0)$, was assigned to the purified thrombomodulin. Based on this coefficient, the amount of the thrombomodulin thus purified was calculated from the absorbance for the fractions containing the purified thrombomodulin, and was found to be about 500 µg. Further, the purified thrombomodulin fractions obtained were subjected to SDS-polyacrylamide gel electrophoresis using a 5 to 10 % gradient, and the band was observed after silver staining.

As a result, only a single band was confirmed. The thrombomodulin obtained was frozen and stored at -80 °C.

Part of the purified human thrombomodulin was dialyzed against a 0.1 % (v/v) aqueous SDS solution at room temperature for 16 hours to obtain a sample for the determination of amino acid sequence. The sample was analyzed according to the method of analyzing chemical structure as mentioned before to determine the amino acid sequence. As a result, it was found that the amino acid sequence as from the second amino acid from the N-terminal was as follows.

Pro-Ala-Glu-Pro-Gln-Pro-Gly-Gly-Ser-Gln

Using a part of the purified human thrombomodulin obtained, the amino acid composition was analyzed by means of an amino acid analyzer, Hitachi 835 (manufactured and sold by Hitachi Ltd., Japan), according to the method of Spackman et al [Spackman et al., Anal. Chem., Vol. 30, p.1190 (1958)]. The results are shown in Table 1. Incidentally, the amino acid composition of the thrombomodulin derived from human placenta was calculated from the data reported in literature [H.H. Salem et al, J. Biol. Chem., Vol. 259, p.12246 (1984)], and is presented in Table 1 for comparison. The calculated result did not agree with the analytical result of the amino acid composition of the thrombomodulin of the present invention. This suggests that the thrombomodulin derived from lung according to the present invention is different from that derived from placenta reported in the prior art

literature.

Table 1

| Amino acid | Composition (%) | |
| --- | --- | --- |
| | Present invention | Referential literature* |
| Aspartic acid | 8.1 | 9.1 |
| Threonine | 6.0 | 4.4 |
| Serine | 7.9 | 7.4 |
| Glutamic acid | 6.8 | 12.5 |
| Proline | 6.5 | 5.9 |
| Glycine | 11.0 | 14.1 |
| Alanine | 8.3 | 8.8 |
| Valine | 8.4 | 6.3 |
| Methionine | 1.4 | 1.6 |
| Isoleucine | 5.4 | 3.4 |
| Leucine | 10.4 | 8.8 |
| Tyrosine | 2.8 | 2.8 |
| Phenylalanine | 3.2 | 2.9 |
| Histidine | 2.7 | 2.2 |
| Lysine | 3.6 | 5.0 |
| Arginine | 5.1 | 4.7 |
| Cysteine | 2.5 | not determined |

- 23 -

0239644

* The amino acid composition of the thrombomodulin derived from human placenta was calculated from the data reported in literature [H.H. Salem et al, J. Biol. Chem., Vol. 259, p.12246 (1984)].

Further, the thromlomodulin obtained was examined with respect to molecular weight and stability according to the above-mentioned method for the determination of molecular weight and methods for stability test.  The following results were obtained.

(a)  Molecular weight

The physiologically active substance of the present invention showed stained bands at a position of 105,000 $\pm$ 10,000, and the substances which had moved to the position exhibited the ability to activate protein C.

(b)  Stability

(1) The substance of the present invention (0.006 $A_{280}$/ml), which had been dissolved in 0.02 M Tris-HCl  buffer (pH 7.5) containing 0.1 M NaCl, was heat-treated at 60 °C for 30 minutes.  No decrease in activity was observed.

(2) A buffer containing the substance of the present invention at the same concentration as that in (1) above was heated at 100 °C to determine the time in which 50 % inactivation occurred.  It was found that the time was about 9 minutes.

(3) The substance of the present invention was added to a solution (pH 2) containing 0.1 M NaCl, 0.02 M glycine, 1 mM benzamidine-HCl, 0.1 % (v/v) Triton X-100 and 5 mM $CaCl_2$ to a concentration of the substance of 0.09 $A_{280}$/ml. After 2.5-hour treatment by leaving the thus obtained mixture as it was, about 70 % of the activity remained. On the other hand, when the substance of the present invention was treated with a solution with the same composition except that the pH was 10, no decrease in activity was observed.

(4) The substance of the present invention was treated with 0.02 M Tris-HCl buffer (pH 7.5) containing 1 % (w/v) SDS and 0.1 M NaCl at a concentration of the present substance of 0.007 $A_{280}$/ml at room temperature for 2.5 hours. After the treatment, the resultant buffer was diluted 100 times with 0.02 M Tris-HCl buffer (pH 7.5) containing 0.1 M NaCl and 1 mg/ml bovine serum albumin, and then the activity was immediately determined. No decrease in activity was observed.

(5) The same treatment as that in (4) above was repeated except that 6 M urea was used in place of the 1 % (w/v) SDS, and the effect of the 6 M urea-treatment was examined. No decrease in activity was observed.

(6) The same treatment as that in (4) above was repeated except that 6 M guanidinohydrochloric acid was used in place of the 1 % (w/v) SDS, and the effect of the treatment with 6 M guanidinohydrochloric acid was examined. After the treatment, about 40 % of the activity remained.

(7) The same treatment as that in (4) above was repeated except that 3 M KSCN was used in place of the 1 % (w/v) SDS, and the effect of the treatment with 3 M KSCN was examined. After the treatment, about 40 % of the activity remained.

(8) The same treatment as that in (4) above was repeated except that 2-mercaptoethyl alcohol was used in place of the 1 % (w/v) SDS, and the effect of the treatment with mercaptoethyl alcohol was examined. When the concentration of the mercaptoethyl alcohol used was 0.1 % (w/v), the remaining activity was about 2 %, and when the concentration was 1 % (w/v), the remaining activity was 0 %.

(c) Activity

(1) Protein C-activating activity

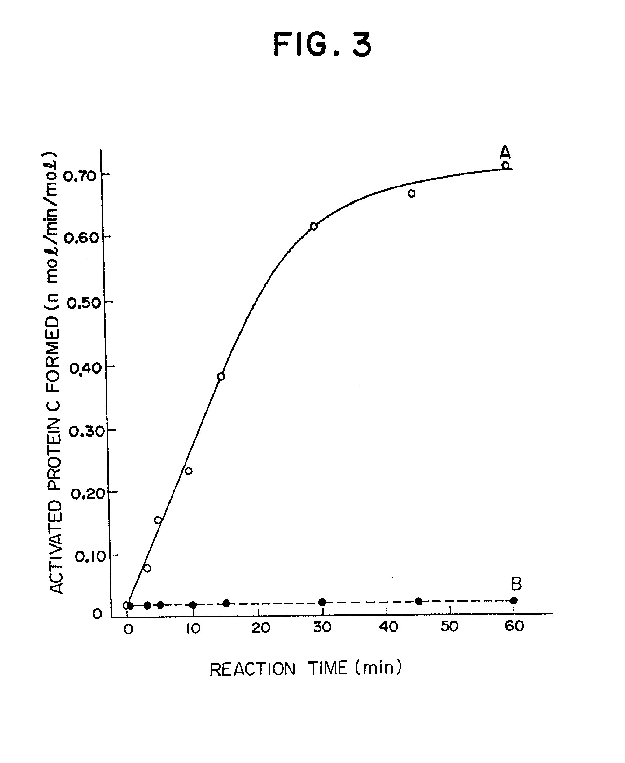

The protein C-activating activity as a cofactor of thrombin was examined according to the method for evaluating activities as mentioned above. The results are shown in Fig. 3. In the case (B) where no thrombomodulin was added, the formation of activated protein C was not observed (represented by the dotted line). In the case (A) where thrombomodulin was added, the amount of protein C was increased with the passage of reaction time (represented by the solid line).

(2) Anticoagulating activity

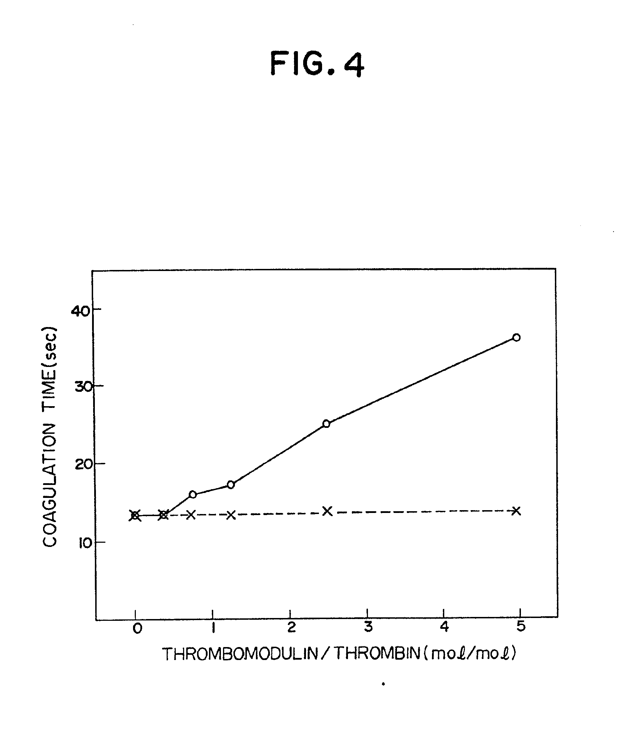

According to the above-mentioned method for evaluating activities, an examination was made with respect to the inhibiting action on the conversion of fibrinogen to fibrin by thrombin which leads to the substantial inhibition of blood coagulation. The results are shown in Fig. 4. In the figure, the dotted line represents the results obtained by the measurement with respect to a sample in which only the buffer was used, and the solid line represents the results obtained by the measurement with respect to the sample to which thrombomodulin was added. It was confirmed that as the amount of thrombomodulin became large relative to that of thrombin, the time taken for the blood coagulation was prolonged.

(3) Platelets aggregation-inhibiting activity

According to the above-mentioned method for evaluating activities, an examination was made with respect to the activity for substantially inhibiting the platelets aggregation-inhibiting activity of thrombin. As a result, when one unit (about 0.4 µg) of thrombin was added to 250 µℓ of a solution containing 300,000 cells/µℓ of blood platelets (Platelet Rich Plasma, P.R.P.), the aggregation of platelets occurred; when, before the addition of thrombin, the thrombomodulin of the present invention was added in a molar amount larger than that of thrombin to be added, the aggrega-

tion of platelets did not occur even after the addition of thrombin.


Application Example 1

| Physiologically active substance of the present invention | 10 mg |
|---|---|
| Purified gelatin | 20 mg |
| Mannitol | 100 mg |
| Sodium chloride | 7.8 mg |
| Sodium phosphate | 15.4 mg |

The above components were dissolved in 2 ml of distilled water for injection. The obtained solution was put in a sterilized vial, preliminarily frozen at -35 °C for 2 hours, and subjected to a first drying at -35 °C at a degree of vacuum of 0.075 Torr for 35 hours, followed by a second drying at 30 °C at a degree of vacuum of 0.03 Torr for 5 hours, thereby to prepare an injection contained in a vial.

The composition thus obtained may be used for intravenous drip infusion. For this purpose, the composition is dissolved in 500 ml of physiological saline or a glucose injection just before the administration.

Application Example 2

Physiologically active substance

of the present invention                 2.5 mg

Albumin                                    5 mg

Mannitol                                  25 mg

Sodium chloride                          1.95 mg

Sodium phosphate                         3.85 mg

Using the above components, an injection contained in a vial was prepared in substantially the same manner as in Application Example 1.


Brief Description of the Drawings

Fig. 1 is a graph showing the elution patterns obtained by the third DIP-thrombin-agarose column chromatography in Example 1.

Fig. 2 is a graph showing the elution patterns obtained by the fourth DIP-thrombin-agarose column chromatography in Example 1.

Fig. 3 is a graph showing the amount of the activated protein C formed with the passage of reaction time in the presence and absence of the thrombomodulin.

Fig. 4 is a graph showing the relationship between the amount of thrombomodulin and the time required for blood coagulation.

Probability of Utilization in Industry

The physiologically active substance of the present invention exhibits at the same time an anti-blood coagulating activity, a platelets aggregation-inhibiting activity and a thrombolytic activity, and is an extremely useful substance as a medicine having few harmful side effects for treating circulatory organ diseases. Further, besides the use as such a medicine, the present substance may be used as a drug which is adapted to be fixed to materials for an artificial blood vessel, an artificial organ and the like to prevent the formation of thrombosis.

What is claimed is:

1.   A physiologically active substance capable of controlling blood coagulation, which is characterized by the following properties:

(a)    molecular weight:

105,000 ± 10,000 (as measured by SDS-polyacrylamide gel electrophoresis after reduction);

(b)    stability:

(1) stable at 60 °C for 30 min,

(2) not inactivated at 100 °C for 10 min,

(3) stable at pH2 to 10,

(4) stable with 1 % (v/v) SDS treatment,

(5) stable with 6 M urea treatment,

(6) not inactivated with 6 M guanidine hydrochloride,

(7) not inactivated with 3 M KSCN treatment, and

(8) inactivated with 1 % (v/v) 2-mercaptoethanol treatment;

(c)    activities:

(1) it activates protein C as a cofactor of thrombin,

(2) it inhibits the conversion of fibrinogen to fibrin by thrombin, thereby substantially inhibiting blood coagulation, and

(3) it substantially inhibits the platelets aggregation activity of thrombin; and

(d)    chemical structure:

it contains a peptide having the following amino acid sequence as from the second amino acid from the amino terminal

Pro-Ala-Glu-Pro-Gln-Pro-Gly-Gly-Ser-Gln

(wherein Pro represents a proline residue, Ala an alanine residue, Glu a glutamic acid residue, Gln a glutamine residue, Gly a glycine residue and Ser a serine residue).

1/4

FIG. 1

FRACTION NUMBER (2mℓ / fraction)

# FIG. 2

FIG. 3

3/4

0239644

# FIG. 4

# INTERNATIONAL SEARCH REPORT

International Application No. PCT/JP86/00330

## I. CLASSIFICATION OF SUBJECT MATTER (if several classification symbols apply, indicate all) [3]

According to International Patent Classification (IPC) or to both National Classification and IPC

Int.Cl[4] A61K35/42, A61K37/02, C07K15/06

## II. FIELDS SEARCHED

| Minimum Documentation Searched [4] | |
|---|---|
| Classification System | Classification Symbols |
| IPC | A61K35/42, A61K37/02, C07K15/06 |

| Documentation Searched other than Minimum Documentation to the Extent that such Documents are Included in the Fields Searched [5] |
|---|
| |

## III. DOCUMENTS CONSIDERED TO BE RELEVANT [14]

| Category* | Citation of Document, [16] with indication, where appropriate, of the relevant passages [17] | Relevant to Claim No. [18] |
|---|---|---|
| | | |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search [2] | Date of Mailing of this International Search Report [2] |
|---|---|
| September 22, 1986 (22.09.86) | October 6, 1986 (06.10.86) |
| International Searching Authority [1] | Signature of Authorized Officer [20] |
| Japanese Patent Office | |

Form PCT/ISA/210 (second sheet) (October 1981)